# FASCICULE DE BREVET EUROPEEN

(11) **EP 0 623 822 B1**
(45) Date de publication et mention de la délivrance du brevet: **01.09.1999**
(21) Numéro de dépôt: 94201130.5
(22) Date de dépôt: 25.04.1994
(51) Int. Cl.: G01N 33/58, G01N 33/543

(54) **Réactifs indicateurs pour la détection ou le dosage d'un analyte, trousses les contenant, et procédé de détection ou de dosage**
Indikatorreagenzien zum Nachweis oder zur Messung von Analyten, Testsatz, der diese enthält, und Verfahren zum Nachweis oder zur Messung
Indicator reagents for detecting or measuring an analyte, kit containing the same and method of detection or measuring

(30) Priorité: 03.05.1993 BE 9300449
(43) Date de publication de la demande: 09.11.1994
(73) Titulaire: DIAgAM, 7822 Ghislenghien (BE)
(72) Inventeur: Englebienne, Patrick, B-9750 Zingem (BE)
(74) Mandataire: Bairiot-Delcoux, Mariette

(56) Documents cités:
- EP-A- 0 227 173
- EP-A- 0 360 088
- WO-A-91/06887
- J.Immunological Methods, vol.191, p.159-170, (1996)
- Chemical Communications, pages 1651-1652, (1996)

## Description

L'invention a pour objet des réactifs utilisables dans différents tests de détection ou de dosage d'analytes, notamment des tests immunologiques, qui mettent en oeuvre des polymères conducteurs, un procédé pour la détection ou le dosage d'un analyte avec ces réactifs et des trousses les contenant.

Depuis les années 80, de nombreuses recherches ont été effectuées dans le domaine des polymères conducteurs, qui sont des polymères organiques bien connus pour leurs intéressantes propriétés optiques et électriques.

Les principaux polymères conducteurs connus sont la poly(aniline), le poly(pyrrole), le poly(thiophène), le poly(acétylène), le poly(phénylène), le poly(naphtalène), le poly(carbazole), le poly(pyrène), le poly(pyrénylène vinylène), le poly(azulène), le poly(isothianaphtène), le poly(phénylène vinylène), le poly(thiophénol), etc...

D'autres polymères non repris ici répondent également à l'appellation de polymères conducteurs.

Tous les polymères conducteurs ont une caractéristique commune : ils contiennent des alternances d'une liaison simple et d'une liaison double entre atomes de carbone le long de leur chaîne polymérique.

Ces polymères sont généralement rendus conducteurs par dopage à l'aide d'un agent extérieur. Le squelette du polymère dopé est réduit ou oxydé, ce qui introduit des charges électroniques mobiles sur la chaîne polymérique, lesquelles charges sont responsables de la conductivité.

On connaît également des polymères conducteurs naturellement auto-dopés.

Un même polymère conducteur peut présenter différentes couleurs suivant son état d'oxydation; ceci provient d'importants changements dans les spectres d'absorption électronique se produisant en fonction des modifications de charge électrique dans le polymère.

La solubilité dans différents milieux de polymères conducteurs et de dérivés de ceux-ci a fait l'objet de nombreuses études.

Dans l'article de J.A. Frommer intitulé "Conducting polymer solutions", Acc. Chem. Res. 1986, 19, 2-9, on décrit par exemple la solubilité d'un polymère conducteur dans des solvants inhabituels tels que du AsF₃, du SbCl₃ ou de l'iode liquide.

Dans l'article de Bryce M.R. et al. intitulé "Soluble, conducting polymers from 3-substituted thiophenes and pyrroles", J. Chem. Soc.; Chem. Commun., 1987, 466-467, on a indiqué que des polymères obtenus par la polymérisation de dérivés de monomères substitués en position 3 par de longues chaînes sont solubles dans les solvants organiques ordinaires.

Récemment, on a réalisé la synthèse de polymères conducteurs solubles dans l'eau.

Dans l'article de Patil et al., intitulé "Self-doped conducting polymers", Synthetic Metals, 20 (1987) 151-159, on mentionne le fait que des dérivés de poly(thiophène) substitués par une chaîne hydrocarbonée relativement longue sur le noyau thiophène ont pu être solubilisés dans l'eau.

Les demandes de brevet WO 91/05979 et WO 91/06887 sont relatives à des dérivés sulfonés hydrosolubles de la poly(aniline).

Dans certaines conditions de synthèse, les polymères conducteurs peuvent en outre être mis en dispersion dans l'eau, et former des solutions colloïdales ou des latex.

Toute technique immunologique visant à détecter ou doser un analyte met en oeuvre la formation de complexes immuns. Ces complexes immuns sont formés par la liaison d'un antigène avec un anticorps. Dans les premiers dosages basés sur ce principe, la formation des complexes immuns est détectée par la turbidité qu'ils provoquent dans le milieu réactionnel. Cette détection s'est progressivement améliorée au cours du temps par l'introduction de traceurs dans les réactifs utilisés, en particulier des traceurs radioactifs, fluorescents, enzymatiques et colloïdaux.

L'intérêt de l'utilisation de particules colloïdales en tant que traceur permettant de suivre la formation de complexes immuns, réside essentiellement dans l'augmentation de la taille de ces complexes que leur présence provoque. La turbidité qui résulte de la formation des complexes "marqués" par ce traceur s'en trouve par conséquent fortement augmentée.

On sait par la demande EP-A-0360088 que des polymères conducteurs ou des dérivés de ceux-ci peuvent être utilisés sous forme de latex dans des réactifs indicateurs, notamment pour des tests immunologiques.

L'utilisation de polymères conducteurs sous forme colloïdale comme traceurs dans des tests immunologiques procède du principe exposé plus haut. Leur utilisation apporte de plus une amélioration par rapport aux colloïdes classiques en ce que ces polymères conducteurs sont en général fortement colorés et donc plus facilement détectables par l'oeil. Cependant, malgré l'intérêt que présente cette technique, les utilisateurs de traceurs colloïdaux sont couramment confrontés à des problèmes d'auto-agglutination et/ou d'interactions non spécifiques dûs à la présence de particules.

Par ailleurs, l'application de polymères solubles dans l'eau en général comme traceurs dans des tests immunologiques n'a pas généré d'intérêt. Ce manque d'intérêt procède de la même logique. En effet, ces polymères hydrosolubles étant de beaucoup plus petite taille, ils ne sont pas susceptibles d'augmenter autant que les polymères colloïdaux, la taille des complexes immuns.

Leur utilisation comme traceurs semblait donc devoir être exclue.

On a à présent montré que l'on peut, contre toute attente, utiliser avec succès, en vue de détecter ou de doser un analyte dans un échantillon, un réactif indicateur selon l'invention, qui comprend un traceur attaché à un partenaire de liaison spécifique, ce traceur étant un polymère conducteur soluble dans l'eau. Les réactifs indicateurs de l'invention sont aptes à émettre un signal lors de la liaison de leur partenaire de liaison spécifique à un partenaire complémentaire. Pour la bonne compréhension de la description de l'invention, quelques définitions sont données ci-après.

Dans le cadre de l'invention, un polymère est défini comme étant une molécule organique formée par la condensation répétitive de molécules plus petites appelées monomères. Lorsqu'un polymère est formé de monomères identiques entre eux, il est appelé homopolymère. Lorsqu'il est formé de monomères différents condensés en alternance, il appelé copolymère. Enfin, lorsque le polymère est de bas poids moléculaire, il est appelé oligomère.

Par "polymère conducteur", on entend tout polymère organique susceptible de montrer des propriétés de conductivité électrique.

Le terme "analyte" est utilisé pour désigner une substance à détecter ou à doser dans un échantillon à tester.

Par "partenaire de liaison spécifique", on entend une molécule faisant partie d'une paire de molécules différentes qui peuvent se lier spécifiquement entre elles par des moyens chimiques ou physiques. Deux partenaires d'une même paire sont dits "complémentaires".

Un exemple frappant de telles paires spécifiques est la paire antigène-anticorps. D'autres paires spécifiques sont par exemple les séquences nucléotidiques complémentaires, les séquences peptidiques complémentaires, les paires haptène-anticorps, les paires récepteur-ligand, les paires enzyme-substrat etc ...

Les réactifs indicateurs de l'invention peuvent être utilisés dans différents types d'immunodosages.

Entre autres, les tests immunologiques auxquels l'invention peut être appliquée sont soit hétérogènes, soit homogènes.

Les tests homogènes sont ceux pour lesquels les composants de la réaction ou les réactifs restent en solution et ne sont pas séparés avant la mesure du signal produit par le réactif indicateur.

Les tests hétérogènes sont ceux pour lesquels une séparation des fractions libres et des fractions liées des composants de réaction ou des réactifs est effectuée avant la mesure du signal. Cette séparation peut être réalisée soit en fixant au préalable le réactif sur une phase solide, soit en précipitant les complexes immuns formés au cours de la réaction à l'aide de moyens physiques, chimiques ou biochimiques (par addition d'un ou de partenaires de liaison accessoires, qui sont spécifiques d'un ou de plusieurs des partenaires de la liaison spécifique principale), et en enlevant ensuite la phase solide ou le précipité du milieu réactionnel par lavage, centrifugation ou filtration.

Les tests homogènes et hétérogènes peuvent également être conçus sur base d'un modèle sandwich ou de compétition.

Ils peuvent également être conçus sur base de modèles direct ou indirect, le modèle indirect mettant en oeuvre un ou des partenaires de liaison spécifique accessoires.

Les indications données ci-dessus ne sont pas restrictives et l'homme de l'art pourra aisément concevoir d'autres types de tests qui ne rentrent pas nécessairement dans les modèles décrits.

Toutefois, l'invention ne se limite pas à cette application. Elle concerne d'une manière générale tous les types de dosage dans lesquels peuvent intervenir des partenaires de liaison spécifique et au moins un traceur consistant en un polymère conducteur hydrosoluble.

Les analytes dosés peuvent être non seulement des antigènes ou des anticorps mais aussi toute substance à laquelle le principe des dosages et des tests de détection réalisés suivant l'invention peut être appliqué.

Par "réactif de capture", on entend un réactif qui est un partenaire de liaison spécifique de l'analyte et qui permet soit d'immobiliser ce dernier (test hétérogène), soit encore d'offrir des sites de liaison pour lesquels l'analyte pourra entrer en compétition avec le réactif indicateur de l'invention (test de compétition).

Par "partenaire de liaison spécifique principal", on entend un partenaire de liaison spécifique tel que défini ci-dessus, qui intervient dans la réaction principale du test de détection ou de dosage. En d'autres termes, son partenaire complémentaire est soit l'analyte, soit le réactif de capture (dans le cas d'un test en compétition).

Par "partenaire de liaison spécifique accessoire", on entend un partenaire de liaison spécifique qui intervient dans une réaction secondaire du test de détection ou de dosage (test indirect ou test hétérogène) et qui a comme partenaire complémentaire par exemple le partenaire de liaison spécifique principal, ou encore le réactif de capture, leur complexe avec l'analyte, ou même le traceur lui-même lorsque ce dernier est fixé à l'un ou à plusieurs de ces autres réactifs.

Dans les réactifs selon l'invention, les polymères conducteurs peuvent être des homopolymères ou des copolymères formés par condensation de dérivés substitués de monomères choisis dans le groupe comprenant l'aniline, le pyrrole, le thiophène, l'acétylène, le phénylène, le naphtalène, le carbazole, le pyrène, le pyrénylène vinylène, l'azulène, l'isothianaphtène, le phénylène vinylène et le thiophénol.

De préférence, le polymère conducteur est formé de monomères subtitués par un ou plusieurs groupements choisis parmi les groupements carboxylique, sulfonique, phénol, thiol, alcool, aldéhyde, amine, nitrile, amide, cétone, nitré et halogénés.

Chaque groupement de substitution peut être fixé soit directement sur le monomère, soit indirectement sur celui-ci par l'intermédiaire d'une chaîne hydrocarbonée ou d'un cycle.

Des polymères conducteurs hydrosolubles préférés sont la poly(aniline-2-acide carboxylique), la poly(aniline-2-acide sulfonique), le poly(thiophène-3-acide carboxylique) et le poly(thiophène-3-carboxaldéhyde).

Les réactifs indicateurs de l'invention peuvent se présenter sous forme sèche ou sous forme dissoute dans un milieu aqueux.

Les réactifs indicateurs de l'invention peuvent être associés à un support solide, et dans une forme de réalisation intéressante, se présenter sous forme d'un film. Le signal émis par les réactifs indicateurs de l'invention peut être un signal optique détectable ou quantifiable par l'oeil, il peut être un signal optique détectable ou quantifiable par un instrument.

L'instrument utilisé pour sa détection ou sa quantification peut être apte à détecter ou quantifier la réflexion, la diffraction, la réfraction ou l'absorption lumineuse ultra-violette, visible ou infrarouge.

De préférence, le partenaire de liaison spécifique auquel le traceur du réactif indicateur peut être attaché et son partenaire complémentaire sont choisis dans le groupe consistant en antigènes, haptènes et anticorps, enzymes et substrats, récepteurs et ligands.

Le traceur peut s'attacher à ce partenaire de liaison spécifique par liaison covalente ou par adsorption passive.

Le partenaire de liaison spécifique d'un réactif indicateur selon l'invention peut être selon le cas un partenaire de liaison spécifique principal ou accessoire.

L'invention a également pour objet l'utilisation d'un traceur consistant en un polymère conducteur hydrosoluble, ce traceur étant susceptible de s'attacher à un partenaire de liaison spécifique, et pouvant être contenu dans un réactif indicateur suivant l'invention.

L'invention a également pour objet des trousses pour la détection ou le dosage d'un analyte dans un échantillon à tester, qui contiennent au moins un réactif indicateur selon l'invention. Elles peuvent contenir en outre un réactif de capture. Celui-ci peut, le cas échéant, être fixé sur une phase solide.

Selon une forme d'exécution particulière, ces trousses contiennent en outre au moins un partenaire de liaison spécifique accessoire.

L'invention a également pour objet un procédé pour détecter un analyte dans un échantillon à tester qui comprend les étapes suivantes :
a) introduire dans l'échantillon au moins un réactif indicateur suivant l'invention;
b) laisser ledit réactif indicateur se lier au partenaire complémentaire du partenaire de liaison spécifique qu'il contient;
c) séparer le cas échéant les fractions libres et liées du réactif indicateur;
d) détecter le réactif indicateur et donc la présence de l'analyte dans l'échantillon.
   Suivant une forme d'exécution particulière, le procédé de détection selon l'invention comprend en outre l'étape suivante :
e) introduire dans l'échantillon à tester un réactif de capture, éventuellement fixé sur une phase solide.
   Eventuellement, il comprend encore l'étape suivante :
f) si le partenaire de liaison spécifique du réactif indicateur n'a pas l'analyte comme partenaire complémentaire, introduire dans l'échantillon à tester au moins un partenaire de liaison spécifique principal.
   En outre, le procédé peut encore comprendre l'étape suivante :
g) introduire dans l'échantillon à tester au moins un partenaire de liaison spécifique accessoire.
   Il est bien entendu que les étapes a, e, f et g peuvent se dérouler simultanément ou en séquence.
   L'invention a également pour objet un procédé pour doser un analyte d'un échantillon à tester, dans lequel on détecte l'analyte selon un des procédés décrits ci-dessus,
h) on effectue une mesure du signal et
i) on établit une corrélation entre cette mesure et la quantité d'analyte présent dans l'échantillon.

L'invention sera mieux comprise en se référant aux exemples d'exécution décrits ci-après, et illustrés aux figures, dans lesquelles :
- la figure 1 montre une courbe dose-réponse obtenue lors d'un dosage compétitif de sérum albumine humaine (hSA), utilisant de la poly(aniline) colloïdale (en abrégé PA) comme traceur, dans un réactif selon l'état de la technique;
- la figure 2 montre une courbe dose-réponse obtenue lors d'un dosage compétitif de sérum albumine humaine (hSA) utilisant de la poly(aniline-2-acide carboxylique) (en abrégé PA-COOH) hydrosoluble comme traceur dans un réactif indicateur selon l'invention, dans des conditions de concentration en réactif indicateur et en antisérum comparables à celles utilisées pour la courbe montrée sur la figure 1;
- la figure 3 montre des courbes dose-réponse obtenues avec différentes concentrations en antisérum lors d'un dosage compétitif d'apo-transférrine humaine (hTRF) en utilisant du poly(thiophène-3-acide carboxylique) (en abrégé PT-COOH) hydrosoluble comme traceur dans un réactif indicateur selon l'invention;
- la figure 4 montre des courbes dose-réponse obtenues lors d'un dosage en excès d'anticorps de la protéine C-réactive humaine (h-CRP), en utilisant comme traceur soit du poly(thiophène-3-carboxaldéhyde) (en abrégé PT-CHO) hydrosoluble dans un réactif indicateur selon l'invention, soit l'antisérum non marqué (contrôle);
- la figure 5 montre la courbe dose-réponse obtenue lors d'un dosage en excès d'anticorps de la h-CRP, en utilisant de la poly(aniline-2-acide sulfonique) (en abrégé PA-SO₃) hydrosoluble comme traceur dans un réactif indicateur selon l'invention;
- les figures 6 à 9 illustrent respectivement les effets du pH sur les spectres d'absorption électronique de la PA-SO₃, de la PA-COOH, du PT-COOH et du PT-CHO en solution aqueuse, et
- la figure 10 montre la comparaison des spectres d'absorption électronique de la PA colloïdale et de la PA-SO₃ hydrosoluble en solution aqueuse au même pH.

### Exemples

### 1) Préparation de polymères conducteurs hydrosolubles :

Les synthèses ont été réalisées à partir des monomères substitués suivants :
1) acide aniline-2-sulfonique
2) acide aniline-2-carboxylique
3) acide thiophène-3-carboxylique
4) thiophène-3-carboxaldéhyde
dans un milieu aqueux contenant 8,4 ml d'HCl 37% par 100 ml d'eau, en présence d'un agent oxydant (persulfate d'ammonium).

On a fait réagir les différents monomères avec du persulfate d'ammonium à la température ambiante pendant un temps variable en fonction de la vitesse d'apparition du polymère soit
- 48 heures pour la PA-SO₃;
- 24 heures pour la PA-COOH;
- 3 1/2 heures, pour le PT-COOH;
- 4 1/2 heures, pour le PT-CHO.

Ces conditions de synthèse sont données à titre d'exemples et ne sont pas limitatives.

A la fin de la synthèse, les solutions ont été dialysées contre de l'eau désionisée courante.

A l'exception de l'acide thiophène-3-carboxylique, qui a été dissous dans de l'eau chaude, les monomères ont été laissés à température ambiante.

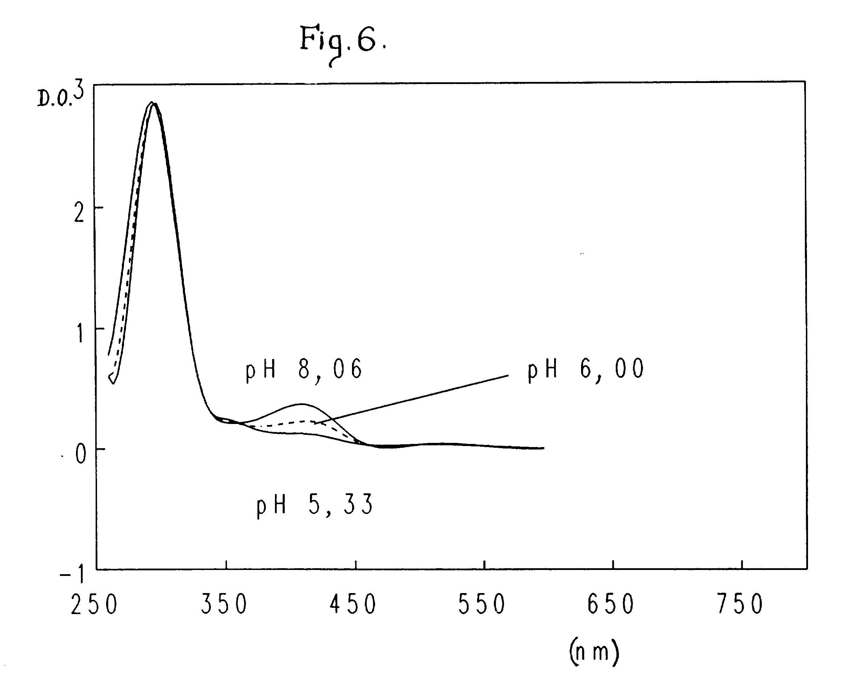

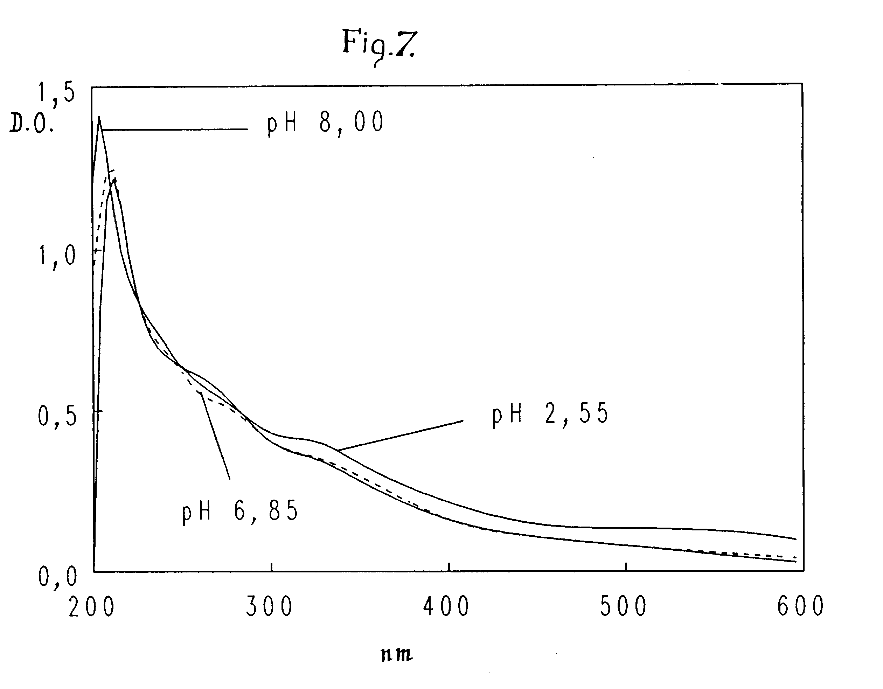

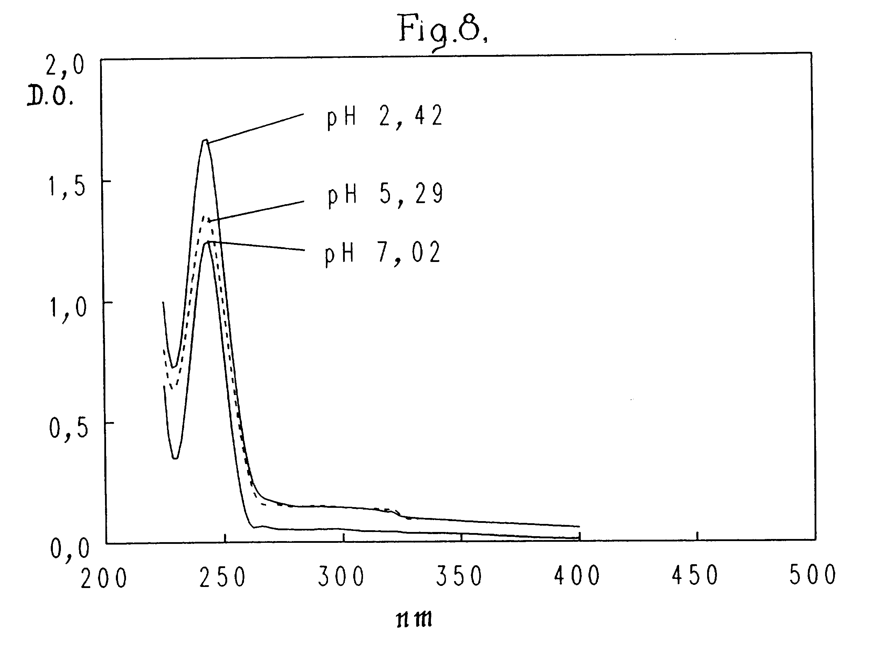

Les spectres d'absorption des différents polymères à différents pH sont illustrés aux figures 6 à 9 commentées plus loin.

A des fins de comparaison, on a également synthétisé de la polyaniline en solution colloïdale (en abrégé PA). De l'alcool polyvinylique (10 grammes;PVA) a été dissous dans 200 ml d'eau bouillante. De l'eau a été ajoutée en permanence pour compenser l'évaporation. La solution encore chaude a été filtrée et une fois refroidie, elle a été acidifiée par addition de 16,8 ml d'HCl 12 N. La moitié a été transférée dans un récipient propre et placée à 4°C sous agitation magnétique.

0,25 ml d'aniline ont été injectés dans la solution et celle-ci a été laissée à refroidir.

0,658 g de persulfate d'ammonium dissous dans 5 ml d'eau ont été ajoutés et on a laissé la polymérisation s'effectuer pendant 48 heures à 4°C. La solution a été extensivement dialysée contre de l'eau désionisée et le colloïde a été lavé par centrifugations successives.

### 2) Chromatographie

Des colonnes (2x40 cm) sont remplies avec du Séphacryl S-1000 et équilibrées avec du tampon phosphate 0,1 M à pH 8.0. Des aliquotes de solution aqueuse de PA-SO₃, PA-COOH, PT-COOH et PT-CHO et de solution colloïdale de PA (dans ce dernier cas, le tampon contient en outre 0,5% v:v de Tween 20) sont chargées sur les colonnes et chromatographiées en utilisant le tampon d'équilibration comme éluant. Dans chaque cas, les fractions correspondant au premier pic contenant le polymère sont récoltées et conservées à la température ambiante.

Les différents polymères hydrosolubles restent stables au moins 24 heures après la synthèse. Leur stabilité est de plusieurs semaines au moins après purification par chromatographie. Leur poids moléculaire était compris après chromatographie, entre 50 et 100 kDa.

L'état de solution vraie des solutions aqueuses de polymères hydrosolubles a été vérifié sur base des critères suivants : on n'observe pas de formation de culot après centrifugation pendant deux heures à 45.000 g et l'étude de l'émission de photons par diffraction de lumière étudiée à angle variable est statistiquement comparable à celle du bruit de fond.

### 3) Couplages

Les différents polymères conducteurs hydrosolubles synthétisés sont utilisés comme traceurs dans des réactifs indicateurs pour tests de détection ou de dosage. Ces traceurs doivent être préalablement attachés à un partenaire de liaison spécifique. Dans les exemples donnés ci-dessous, ces partenaires de liaison spécifiques sont des protéines, en particulier des antigènes ou des anticorps.

Des couplages ont donc été réalisés après purification par chromatographie, soit par liaison covalente, soit par adsorption passive, selon des protocoles différents en fonction de la structure du polymère utilisé.

Pour la PA-COOH et le PT-COOH, on a utilisé une carbodiimide comme agent de couplage.

Le couplage du PT-CHO avec des protéines a lieu par formation d'aldimine.

Le couplage du PA-SO₃ avec les protéines s'effectue par adsorption passive.

### a) Couplage covalent de sérum albumine humaine (hSA) avec de la poly(aniline) en solution colloïdale (PA)

40 ml du polymère purifié par chromatographie ont été mélangés à 20 ml de glutaraldéhyde 25% (v:v) et incubés pendant 24 heures à 37°C. Ensuite, le mélange est de nouveau purifié par chromatographie, les fractions correspondant au pics de polymère étant collectées.

10 mg de hSA par ml ont été ajoutés à la solution, et le mélange a été incubé pendant 48 heures à 4°C. De l'éthanolamine a été ajoutée jusqu'à obtention d'une solution molaire, et le mélange a été de nouveau incubé pendant 48 heures à 4°C puis repurifié par chromatographie.

### b) Couplage covalent de hSA avec la poly (aniline-2-acide-carboxylique) (PA-COOH) en solution aqueuse

30 mg de chlorhydrate de 1-3-diméthylaminopropyl)-3-éthyl carbodiimide sont ajoutés à 30 mg de hSA dissous dans 25 ml d'eau. 30 ml de solution de poly(aniline-2-acide-carboxylique) (4,4 U.A./ml à 325 nm et pH 6.85) purifiés par chromatographie ont été ajoutés goutte à goutte sous agitation magnétique à la solution de protéine, le pH étant ajusté et maintenu à 5,5 avec de HCl 0,1N.

Après incubation à la température ambiante pendant 10 minutes, 10 mg du dérivé de carbodiimide ont été de nouveau ajoutés et le mélange a été conservé dans l'obscurité sous agitation magnétique, pendant 18 heures. La solution a été concentrée jusqu'à 25 ml par dialyse contre de l'Aquacid III et puis diluée jusqu'à 30 ml avec du tampon phosphate 0,1 M pH 8.0 et ajustée à pH 7.0 avec du NaOH 6M.

### c) Couplage covalent de hTRF avec le poly(thiophène-3-acide carboxylique) (PT-COOH) en solution aqueuse

Le protocole suivi est similaire à celui décrit sous b).

### d) Couplage covalent d'anticorps anti-hCRP avec le poly(thiophène-3-carboxaldéhyde) (PT-CHO) en solution aqueuse

1 ml de solution tamponnée de la fraction IgG de l'antisérum de lapin a été mélangé avec 0,3 ml de polymère purifié par chromatographie et le mélange a été incubé pendant 48 heures à 4°C, puis utilisé sans autre traitement.

### e) Couplage passif d'anticorps anti-hCRP avec la poly(aniline-2-acide sulfonique) (PA-SO₃) en solution aqueuse

De l'antisérum de chèvre anti-hCRP a été mélangé en quantités égales avec le polymère purifié par chromatographie et le mélange a été incubé pendant 48 heures à 4°C, puis utilisé sans autre traitement.

### 4) Dosages

### 4.1. Immunodosages compétitifs (avec excès d'antigène)

Dans de tels dosages, l'analyte à doser entre en compétition avec une quantité fixée d'analyte marqué par le traceur, pour une quantité fixée de sites de liaison d'un anticorps.

### 4.1.a) Dosage en compétition de hSA en utilisant comme traceur de la poly(aniline) en solution colloïdale (PA)

La présence de hSA sur les particules de PA a été vérifiée par analyse du spectre d'absorption électronique, un pic à 280 mm étant indicateur de la présence de la protéine dans le produit du couplage.

Le réactif PA-hSA résultant du couplage a été utilisé pour titrer l'antisérum dilué dans un tampon imidazole 35 mM pH 7.0, contenant 150 mM de NaCl et 40g/l de poly(éthylène glycol) 6000. Une dilution d'anticorps fournissant un signal situé entre la moitié et le tiers de celui généré pour une dilution d'anticorps saturant la quantité de traceur utilisée a été sélectionnée pour le dosage.

Ce dosage a été effectué par un analyseur automatique Cobas Mira pour lequel un programme a été écrit au préalable. Ce programme prévoit que 30 µl de traceur et 30 µ d'échantillon (hSA diluée dans du NaCl 0.15 M) soient mélangés à 300 µl de la dilution d'anticorps. Après une incubation de 10 minutes à 37°C, la différence de densité optique lue à 340 nm entre le moment de la fin de l'incubation et le moment du mélange des réactifs et de l'échantillon est calculée, la mesure de densité optique en fin d'incubation étant enregistrée directement sans qu'il y ait séparation des fractions liées et libres du milieu réactionnel.

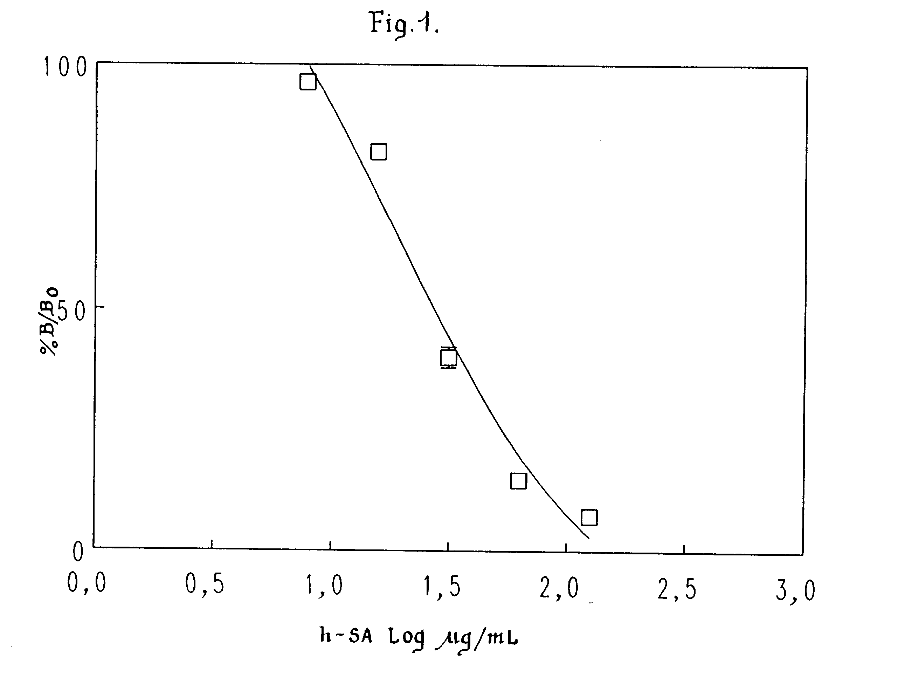

Les résultats obtenus sont illustrés à la figure 1.

Cette figure porte en abscisse le logarithme de la concentration de l'analyte (hSA), en µg/ml, et en ordonnée le pourcentage de B/Bo, où
- Bo est la quantité maximale de réactif (hSA liée au traceur) qui peut se fixer à l'anticorps (anti-hSA) quand le milieu ne contient pas d'analyte (hSA)
- B est la quantité de réactif qui peut se fixer aux sites d'anticorps non occupés par l'analyte présent dans l'échantillon.

La différence de densité optique est convertie en % de B/Bo par un calcul habituel.

### 4.1.b) Dosage en compétition de hSA en utilisant comme traceur de la poly(aniline-2-acide carboxylique) (PA-COOH) hydrosoluble

Le protocole expérimental est le même que pour la solution colloïdale de poly(aniline).

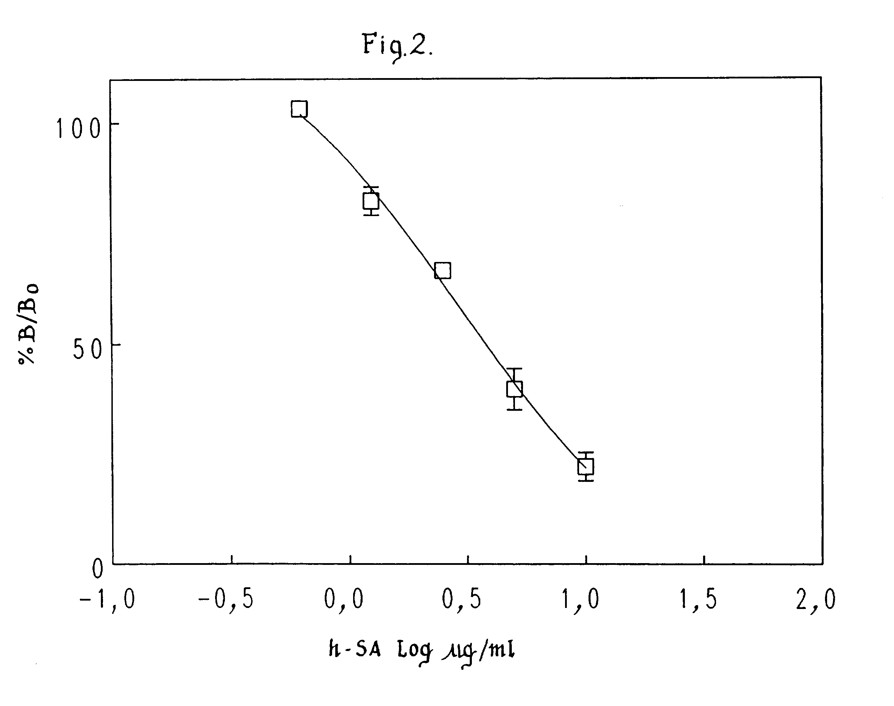

Les résultats obtenus sont illustrés à la Fig. 2, dont les coordonnées sont les mêmes que celles de la Fig. 1.

On voit que pour la même dilution en anticorps et une concentration en traceur similaires à celles utilisées en a), on observe une sensibilité environ dix fois plus élevée.

### 4.1.c) Dosage en compétition de hTRF en utilisant comme traceur du poly(thiophène-3-acide carboxylique) (PT-COOH) hydrosoluble.

Le protocole expérimental est le même que dans les cas a) et b). L'anticorps utilisé est l'anti-hTRF.

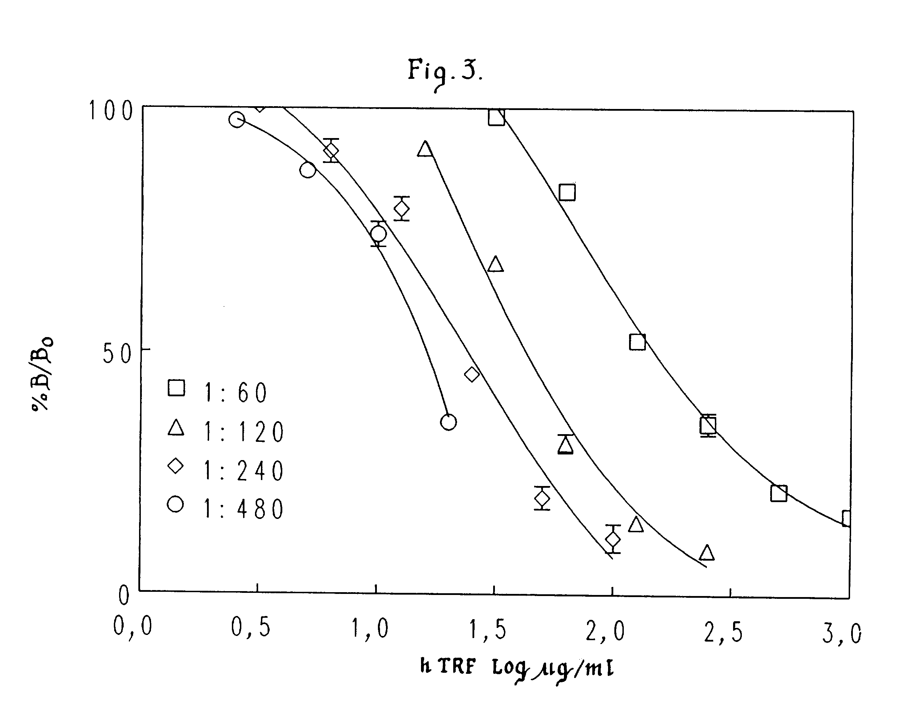

Les résultats obtenus avec différentes dilutions d'antisérum sont illustrés à la figure 3 (mêmes coordonnées que pour les Figs. 1 et 2).

On voit que pour une même activité spécifique du réactif, une dilution progressive de l'antisérum permet d'obtenir une sensibilité de plus en plus importante.

L'activité spécifique est le rapport entre la quantité de traceur et la quantité de partenaire de liaison spécifique dans le réactif.

### 4.2. Immunodosages en excès d'anticorps

Ce type de dosage consiste à incuber l'analyte avec un excès important d'anticorps, puis à enregistrer la densité optique résultant de la formation du complexe immun et à corriger par rapport au blanc.

Les dosages donnés en exemples ci-dessous ont été réalisés sur un analyseur automatique Cobas Mira. Le programme prévoit une dilution 1:4 (v:v) dans du NaCl 150 mM de l'échantillon à doser (sérum humain) et le mélange de 30 µl de cette dilution avec 120 µl de tampon MES 35 mM pH 6.5 contenant 150 mM de NaCl et 40 g/L de poly(éthylène glycol) 6000 et 3 µl de réactif (anticorps dans le cas du dosage témoin et anticorps couplé au traceur dans les autres cas). Après une incubation de 10 minutes, la différence de densité optique est calculée entre la fin de l'incubation et le moment du mélange.

### 4.2.a) Dosage de hCRP en utilisant comme traceur du poly(thiophène-3-carboxaldéhyde) (PT-CHO) hydrosoluble

Dans le cas du dosage avec traceur (PT-CHO) comme dans le cas du dosage témoin (contrôle sans traceur), la densité optique a été enregistrée à 340 nm.

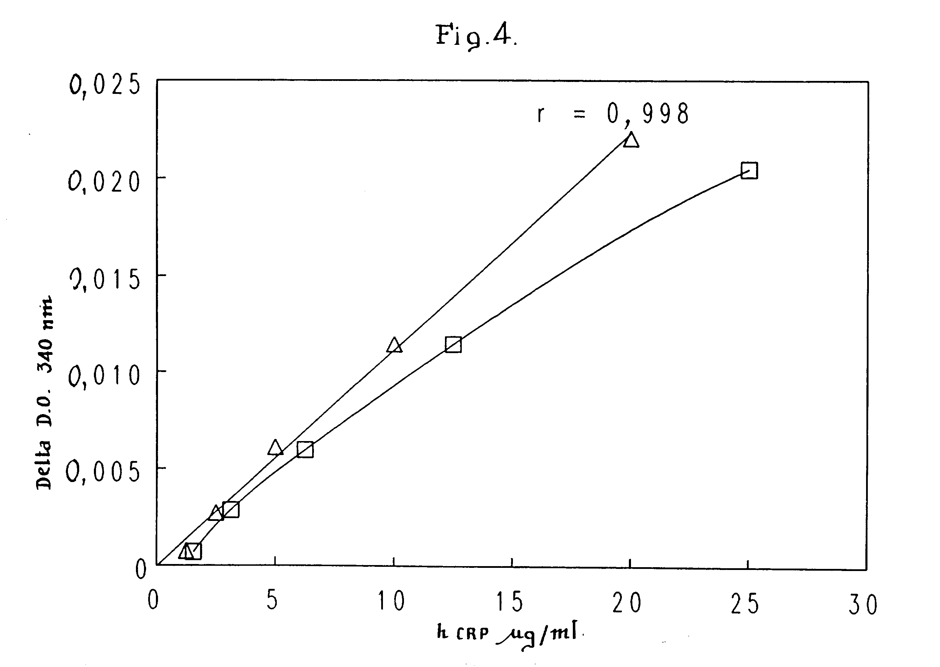

Les résultats sont illustrés à la figure 4, qui porte en ordonnée la différence de densité optique à 340 nm entre l'échantillon et le blanc, et en abscisse la concentration de l'analyte en µg/ml.

Sur cette figure, les résultats obtenus avec le réactif de l'invention du PT-CHO comme traceur sont symbolisés par des triangles, les carrés symbolisant les résultats obtenus avec le témoin (anticorps seul).

On voit que dans le cas où le réactif de l'invention est utilisé, on obtient une relation dose-réponse linéaire ainsi qu'une augmentation sensible du signal par rapport au témoin.

### 4.2.b) Dosage de h-CRP en utilisant comme traceur de la poly(aniline-2-acide sulfonique) (PA-SO₃) hydrosoluble

Dans ce cas, la densité optique a été enregistrée à 405 nm.

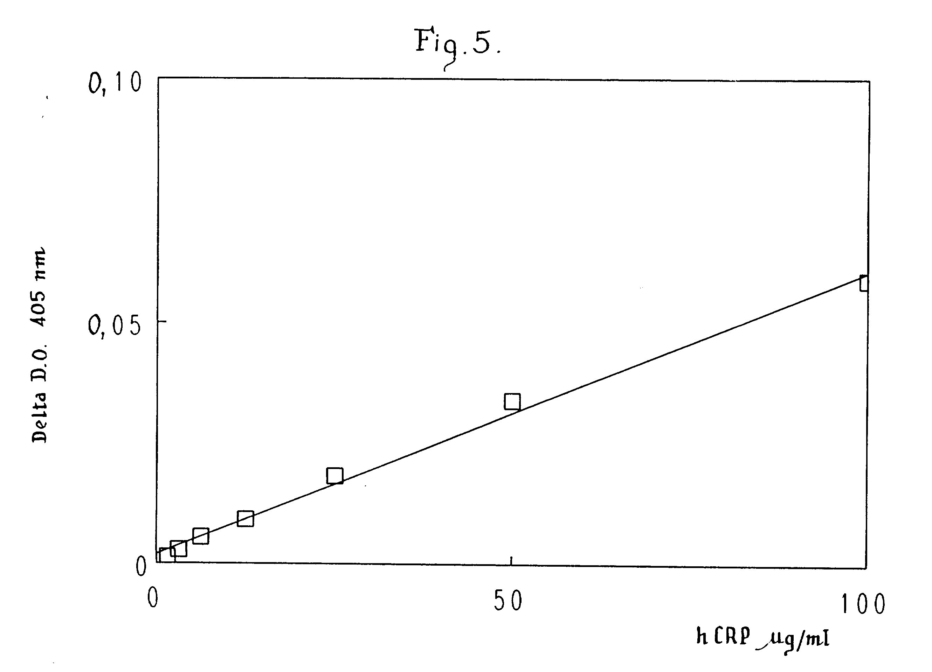

Les résultats sont illustrés à la figure 5, qui porte en ordonnée la différence de densité optique à 405 nm entre l'échantillon et le blanc, et en abscisse la concentration de l'analyte en µg/ml.

On voit que dans ce cas également, on obtient une relation dose-réponse linéaire bien qu'un antisérum non purifié ait été utilisé.

### 5) Analyse spectrale

L'effet du pH sur le spectre des quatre polymères conducteurs hydrosolubles synthétisés est montré respectivement aux figures 6 à 9.

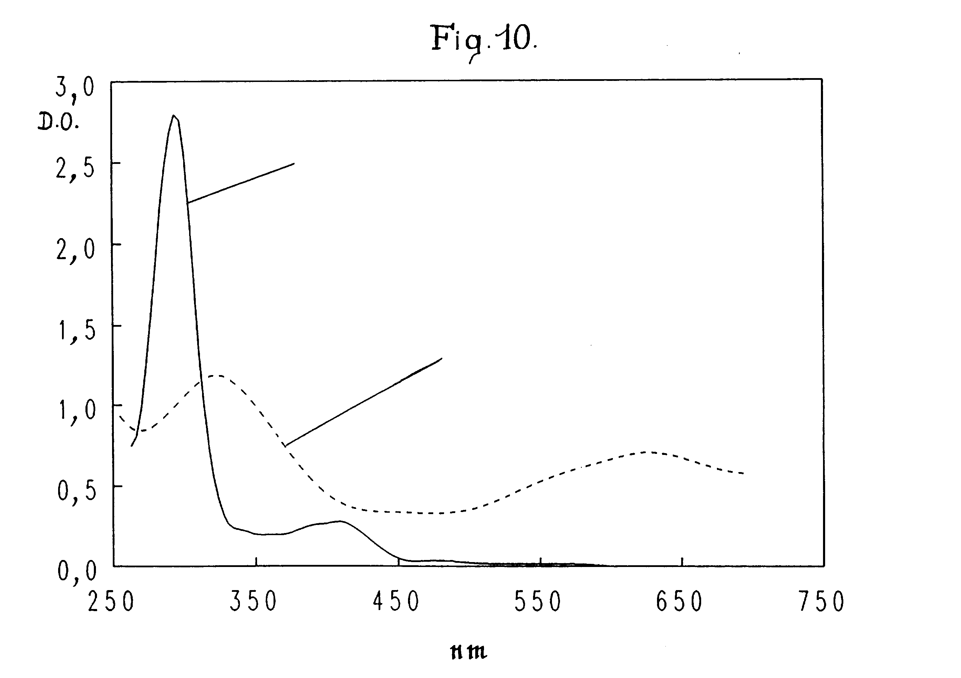

Les graphiques de ces figures et de la figure 10 ont tous comme abscisse la longueur d'onde exprimée en nm, et comme ordonnée la densité optique.

Les solutions de polymères purifiés par chromatrographie ont été diluées de 1:10 à 1:100 (v:v) selon le polymère.

Le pH a été modifié en ajoutant de l'HCl 3 M ou du NaOH 6M. Le volume final de chaque préparation a été ajusté à 10,1 ml avec de l'eau et les spectres ont été enregistrés contre l'air.

On voit que la variation de pH, en modifiant l'état de protonation et donc d'oxydation des polymères, provoque des modifications sensibles dans leurs spectres d'absorption électronique.

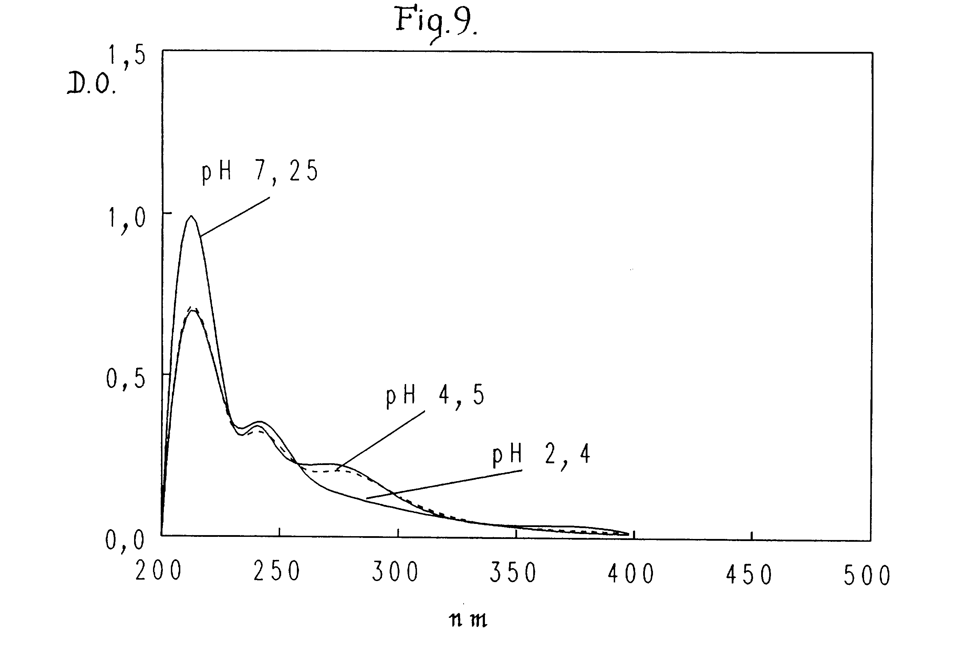

Les spectres superposés sont caractérisés par des maxima :
- de 275 nm (4,5 eV) à 350 nm (3,5 eV) et 410 nm (3,02 eV) pour le PASO₃ (Figure 6);
- à 210 nm (5,76 eV), 225 nm (5,38 eV), 275 nm (4,50 eV) et 320 nm (3,87 eV) pour PA-COOH (Figure 7);
- à 245 nm (5,06 eV) et 310 nm (3,99 eV) pour le PT-COOH (Figure 8);
- à 220 nm (5,63 eV), 240 nm (5,16 eV) et 275 nm (4,50 eV) pour le PT-CHO (Figure 9).

Suite aux modifications de pH des solutions, les spectres de PA-SO₃, du PA-COOH et du PT-CHO présentent deux points isosbestiques, ce qui indique que deux formes d'oxydo-réduction différentes peuvent se former pour un même polymère.

La figure 10 montre la comparaison des spectres d'absorption de la poly(aniline) colloïdale (trait pointillé) et de la poly(aniline-2-acide sulfonique) (trait continu) au même pH.

Dans les deux cas, la bande de haute énergie (4,5 eV pour PA-SO₃ et 3,85 eV pour PA) est due à la transition pi-pi* tandis que la bande d'énergie plus faible (3,02 eV pour PA-SO₃ et 1,98 eV pour PA) est attribuée à l'absorption du polaron (radical ion). Dans le cas de la PA-SO₃, on observe un glissement hypsochrome des deux bandes par rapport au spectre de PA. Ce glissement, parfaitement conforme aux données de la littérature, est expliqué par la différence de conformation du polymère substitué par rapport au polymère non substitué. La PA est en conformation plane, alors que la présence d'un substituant sur les cycles de PA-SO₃ induit une interaction stérique amenant une conformation non plane. Celle-ci réduit la longueur effective de conjugaison entre monomères, ce qui provoque un glissement vers le bleu des bandes d'absorption électroniques.

### 6) Interprétation

Le signal généré par le réactif indicateur suite à la liaison des partenaires de liaison spécifique complémentaires ne résulte pas uniquement de la turbidité provoquée par l'augmentation de la taille des complexes formés. Il est vraisemblable qu'il résulte également de la transduction directe en un signal optique par le polymère des subtiles modifications de charges électriques dans le microenvironnement entourant la poche de liaison (par exemple lors de la fixation d'un antigène à un anticorps dans le cas d'un test de dosage immunologique).

Ceci permet d'expliquer que l'on puisse obtenir un signal photométrique amélioré bien que la taille des complexes (par exemple : complexes immuns) formés ne soient pas sensiblement augmentée, contrairement à ce qui se passe lors de l'utilisation de polymères sous forme colloïdale.

On a donc montré que des polymères conducteurs hydrosolubles peuvent être utilisés avec succès dans des dosages avec excès d'anticorps, ou avec excès d'antigène (tests compétitifs) et qu'ils produisent un signal optique proportionnel à la dose de l'analyte testé, signal lisible sans qu'il soit nécessaire de séparer les fractions libres des fractions liées, et ce avec une très bonne sensibilité.

Grâce à la présence de substituants sur les cycles ou les chaînes, il est possible de coupler ces polymères conducteurs hydrosolubles de façon covalente ou non à de nombreuses molécules.

Les polymères conducteurs hydrosolubles peuvent en outre être utilisés à des pH physiologiques et trouvent de ce fait de nombreuses applications en biochimie.

L'utilisation de ces polymères conducteurs hydrosolubles dans des réactifs selon l'invention fournit une technique analytique homogène, permettant une mesure de signal directe, et dans certains cas, permettant également d'obtenir une relation dose-réponse linéaire.

Grâce à la grande homogénéité que l'on peut obtenir dans les solutions aqueuses, les polymères conducteurs hydrosolubles présentent des avantages certains par rapport aux mêmes polymères en solution colloïdale, notamment du point de vue de la processibilité.

Ceci est d'une importance considérable pour la conception de produits sous forme de films ou encore de senseurs biochimiques.

Les résultats expérimentaux donnés ci-dessus sont tous relatifs à des dosages.

Toutefois, on peut utiliser les réactifs indicateurs de l'invention dans des tests de détection, tels que par exemple des tests de grossesse.

## Revendications

1. Réactif indicateur pour la détection ou le dosage d'un analyte dans un échantillon à tester, comprenant un traceur attaché à un partenaire de liaison spécifique, et apte à émettre un signal lors de la liaison du partenaire de liaison specifique à un partenaire complémentaire, caractérisé en ce que le traceur est un polymère conducteur soluble dans l'eau.

2. Réactif suivant la revendication 1, caractérisé en ce que le polymère conducteur est un homopolymère ou un copolymère formé par condensation de dérivés substitués de monomères choisis dans le groupe comprenant l'aniline, le pyrrole, le thiophène, l'acétylène, le phénylène, le naphtalène, le carbazole, le pyrène, le pyrénylène vinylène, l'azulène, l'isothianaphtène, le phénylène vinylène et le thiophénol.

3. Réactif suivant l'une quelconque des revendications 1 et 2, caractérisé en ce que le polymère conducteur est formé de monomères substitués par un ou plusieurs groupements choisis parmi les groupements carboxylique, sulfonique, phénol, thiol, alcool, aldéhyde, amine, nitrile, amide, cétone, nitré et halogénés.

4. Réactif suivant l'une quelconque des revendications 2 et 3, caractérisé en ce qu'un ou plusieurs des groupements de substitution est ou sont fixés directement sur le monomère.

5. Réactif suivant l'une quelconque des revendications 2 à 4, caractérisé en ce qu'un ou plusieurs des groupements de substitution est ou sont fixés indirectement sur le monomère.

6. Réactif suivant l'une quelconque des revendications 2 et 4, caractérisé en ce que le polymère conducteur soluble dans l'eau est la poly(aniline-2-acide carboxylique).

7. Réactif suivant l'une quelconque des revendications 2 et 4, caractérisé en ce que le polymère conducteur soluble dans l'eau est la poly(aniline-2-acide sulfonique).

8. Réactif suivant l'une quelconque des revendications 2 et 4, caractérisé en ce que le polymère conducteur soluble dans l'eau est le poly(thiophène-3-acide carboxylique).

9. Réactif suivant l'une quelconque des revendications 2 et 4, caractérisé en ce que le polymère conducteur soluble dans l'eau est le poly(thiophène-3-carboxaldéhyde).

10. Réactif suivant l'une quelconque des revendications 1 à 9, caractérisé en ce qu'il est sous forme sèche.

11. Réactif suivant l'une quelconque des revendications précédentes, caractérisé en ce qu'il est associé à un support solide.

12. Réactif suivant la revendication 11, caractérisé en ce qu'il se présente sous forme de film.

13. Réactif suivant l'une quelconque des revendications 1 à 9, caractérisé en ce qu'il est sous forme dissoute dans un milieu aqueux.

14. Réactif suivant l'une quelconque des revendications précédentes, caractérisé en ce que le signal est un signal optique détectable ou quantifiable par l'oeil.

15. Réactif suivant l'une quelconque des revendications précédentes, caractérisé en ce que le signal est un signal optique détectable ou quantifiable par un instrument.

16. Réactif suivant la revendication 15, caractérisé en ce que l'instrument est apte à détecter ou quantifier la réflexion, la diffraction, la réfraction ou l'absorption lumineuse ultra-violette, visible, ou infrarouge.

17. Réactif suivant l'une quelconque des revendications précédentes, caractérisé en ce que le partenaire de liaison spécifique et son partenaire complémentaire sont choisis dans le groupe consistant en antigènes, haptènes et anticorps, enzymes et substrats, récepteurs et ligands.

18. Réactif suivant l'une quelconque des revendications précédentes, caractérisé en ce que le traceur est attaché au partenaire de liaison spécifique par liaison covalente.

19. Réactif suivant l'une quelconque des revendications 1 à 17, caractérisé en ce que le traceur est attaché au partenaire de liaison spécifique par adsorption passive.

20. Réactif suivant l'une quelconque des revendications précédentes, caractérisé en ce que le traceur est attaché à un partenaire de liaison spécifique principal.

21. Réactif suivant l'une quelconque des revendications 1 à 19, caractérisé en ce que le traceur est attaché à un partenaire de liaison spécifique accessoire.

22. Utilisation d'un polymère conducteur soluble comme traceur susceptible de s'attacher à un partenaire de liaison spécifique, et pouvant être contenu dans un réactif indicateur suivant l'une quelconque des revendications 1 à 21.

23. Trousse pour la détection ou le dosage d'un analyte dans un échantillon à tester, caractérisé en ce qu'elle contient au moins un réactif indicateur suivant l'une quelconque des revendications 1 à 21.

24. Trousse suivant la revendication 23, caractérisée en ce qu'elle contient en outre un réactif de capture.

25. Trousse suivant la revendication 24, caractérisé en ce que le réactif de capture est fixé sur une phase solide.

26. Trousse suivant l'une quelconque des revendications 23 à 25, caractérisée en ce qu'elle contient en outre au moins un partenaire de liaison spécifique accessoire.

27. Procédé pour détecter un analyte dans un échantillon à tester, caractérisé en ce qu'il comprend les étapes suivantes :
a) introduire dans l'échantillon au moins un réactif indicateur suivant l'une quelconque des revendications 1 à 21;
b) laisser le réactif indicateur se lier au partenaire complémentaire du partenaire de liaison spécifique qu'il contient;
c) séparer le cas échéant les fractions libres et liées du réactif indicateur;
d) détecter le réactif indicateur et donc la présence de l'analyte dans l'échantillon.

28. Procédé suivant la revendication 27, caractérisé en ce qu'il comprend en outre l'étape suivante :
e) introduire dans l'échantillon à tester un réactif de capture, éventuellement fixé sur une phase solide.

29. Procédé suivant l'une quelconque des revendications 27 et 28, caractérisé en ce qu'il comprend en outre l'étape suivante :
f) si le partenaire de liaison spécifique du réactif indicateur n'a pas l'analyte comme partenaire complémentaire, introduire dans l'échantillon à tester au moins un partenaire de liaison spécifique principal.

30. Procédé suivant l'une quelconque des revendications 27 à 29, caractérisé en ce qu'il comprend en outre l'étape suivante :
g) introduire dans l'échantillon à tester au moins un partenaire de liaison spécifique accessoire.

31. Procédé suivant l'une quelconque des revendications 27 à 30, caractérisé en ce que les étapes a, e, f et g se déroulent simultanément ou en séquence.

32. Procédé pour doser un analyte dans un échantillon à tester, caractérisé en ce qu'il comprend les étapes d'un procédé de détection suivant l'une quelconque des revendications 27 à 31 et les étapes suivantes :
h) on effectue une mesure du signal émis par le réactif indicateur et
i) on établit une corrélation entre cette mesure et la quantité d'analyte présent dans l'échantillon.

## Patentansprüche

1. Indikatorreagenz zum Nachweis oder zur Dosierung eines Analyten in einer zu prüfenden Probe, enthaltend einen an einen spezifischen Bindungspartner gebundenen Tracer, der bei Bindung des spezifischen Bindungspartners an einen Komplementärpartner ein Signal senden kann, dadurch gekennzeichnet, daß es sich bei dem Tracer um ein wasserlösliches leitfähiges Polymer handelt.

2. Reagenz nach Anspruch 1, dadurch gekennzeichnet, daß es sich bei dem leitfähigen Polymer um ein Homo- oder Copolymer handelt, das durch Kondensation substituierter Derivate von Monomeren gebildet wurde, die ausgewählt sind aus der Gruppe bestehend aus Anilin, Pyrrol, Thiophen, Acetylen, Phenylen, Naphthalin, Carbazol, Pyren, Pyrenylen Vinylen, Azulen, Isothianaphthen, Phenylen Vinylen und Thiophenol.

3. Reagenz nach einem der Ansprüche 1 oder 2, dadurch gekennzeichnet, daß die Bildung des leitfähigen Polymers aus Monomeren erfolgt, die durch eine oder mehrere Gruppierungen, ausgewählt aus den Gruppierungen Carboxyl, Sulfonyl, Phenol, Thiol, Alkohol, Aldehyd, Amin, Nitril, Amid, Keton, Nitro und Halogen, substituiert sind.

4. Reagenz nach einem der Ansprüche 2 oder 3, dadurch gekennzeichnet, daß eine oder mehrere der Substitutionsgruppierungen direkt an das Monomer gebunden ist bzw. sind.

5. Reagenz nach einem der Ansprüche 2 bis 4, dadurch gekennzeichnet, daß eine oder mehrere der Substitutionsgruppierungen indirekt an das Monomer gebunden ist bzw. sind.

6. Reagenz nach einem der Ansprüche 2 oder 4, dadurch gekennzeichnet, daß das wasserlösliche leitfähige Polymer Poly(anilin-2-carbonsäure) ist.

7. Reagenz nach einem der Ansprüche 2 oder 4, dadurch gekennzeichnet, daß das wasserlösliche leitfähige Polymer Poly(anilin-2-sulfonsäure) ist.

8. Reagenz nach einem der Ansprüche 2 oder 4, dadurch gekennzeichnet, daß das wasserlösliche leitfähige Polymer Poly(thiophen-3-carbonsäure) ist.

9. Reagenz nach einem der Ansprüche 2 oder 4, dadurch gekennzeichnet, daß das wasserlösliche leitfähige Polymer Poly(thiophen-3-carboxaldehyd) ist.

10. Reagenz nach einem der Ansprüche 1 bis 9, dadurch gekennzeichnet, daß es in Trockenform vorliegt.

11. Reagenz nach einem der vorhergehenden Ansprüche, dadurch gekennzeichnet, daß es auf einem Feststoffträger angeordnet ist.

12. Reagenz nach Anspruch 11, dadurch gekennzeichnet, daß es in Form eines Films vorliegt.

13. Reagenz nach einem der Ansprüche 1 bis 9, dadurch gekennzeichnet, daß es in einem wäßrigen Medium gelöst vorliegt.

14. Reagenz nach einem der vorhergehenden Ansprüche, dadurch gekennzeichnet, daß es sich bei dem Signal um ein optisches durch das Auge nachweisbares oder quantitativ bestimmbares Signal handelt.

15. Reagenz nach einem der vorhergehenden Ansprüche, dadurch gekennzeichnet, daß es sich bei dem Signal um ein optisches durch ein Instrument nachweisbares oder quantitativ bestimmbares Signal handelt.

16. Reagenz nach Anspruch 15, dadurch gekennzeichnet, daß das Instrument zum Nachweis oder zur quantitativen Bestimmung von Reflexion, Beugung, Brechung oder Absorption von ultraviolettem, sichtbarem oder Infrarot-Licht geeignet ist.

17. Reagenz nach einem der vorhergehenden Ansprüche, dadurch gekennzeichnet, daß der spezifische Bindungspartner und dessen Komplementärpartner aus der Gruppe bestehend aus Antigenen, Haptenen und Antikörpern, Enzymen und Substraten, sowie Rezeptoren und Liganden ausgewählt sind.

18. Reagenz nach einem der vorhergehenden Ansprüche, dadurch gekennzeichnet, daß der Tracer an den spezifischen Bindungspartner über eine kovalente Bindung gebunden ist.

19. Reagenz nach einem der Ansprüche 1 bis 17, dadurch gekennzeichnet, daß der Tracer an den spezifischen Bindungspartner mittels passiver Adsorption gebunden ist.

20. Reagenz nach einem der vorhergehenden Ansprüche, dadurch gekennzeichnet, daß der Tracer an einen spezifischen Hauptbindungspartner gebunden ist.

21. Reagenz nach einem der Ansprüche 1 bis 19, dadurch gekennzeichnet, daß der Tracer an einen spezifischen Nebenbindungspartner gebunden ist.

22. Verwendung eines löslichen leitfähigen Polymers als Tracer, der an einen spezifischen Bindungspartner anbindbar ist und in einem Indikatorreagenz nach einem der Ansprüche 1 bis 21 enthalten sein kann.

23. Kit zum Nachweis oder zur Dosierung eines Analyten in einer zu prüfenden Probe, dadurch gekennzeichnet, daß es mindestens ein Indikatorreagenz nach einem der Ansprüche 1 bis 21 enthält.

24. Kit nach Anspruch 23, dadurch gekennzeichnet, daß es außerdem ein Einfangreagenz enthält.

25. Kit nach Anspruch 24, dadurch gekennzeichnet, daß das Einfangreagenz an einer Feststoffphase befestigt ist.

26. Kit nach einem der Ansprüche 23 bis 25, dadurch gekennzeichnet, daß es weiterhin mindestens einen spezifischen Nebenbindungspartner enthält.

27. Verfahren zum Nachweis eines Analyten in einer zu prüfenden Probe, dadurch gekennzeichnet, daß es die folgenden Schritte umfaßt:
a) man gibt in die Probe mindestens ein Indikatorreagenz nach einem der Ansprüche 1 bis 21;
b) man läßt das Indikatorreagenz an den Komplementärpartner des spezifischen Bindungspartners anbinden, den es enthält;
c) man trennt gegebenenfalls die freien und gebundenen Fraktionen des Indikatorreagenz voneinander;
d) man weist das Indikatorreagenz und somit das Vorliegen des Analyten in der Probe nach.

28. Verfahren nach Anspruch 27, dadurch gekennzeichnet, daß es weiterhin folgenden Schritt umfaßt:
e) man gibt in die zu prüfende Probe ein gegebenenfalls an einer Feststoffphase befestigtes Einfangreagenz.

29. Verfahren nach einem der Ansprüche 27 oder 28, dadurch gekennzeichnet, daß es weiterhin folgenden Schritt umfaßt:
f) man gibt in die zu prüfende Probe, falls der Analyt nicht der Komplementärpartner des spezifischen Bindungspartners des Indikatorreagenz ist, mindestens einen spezifischen Hauptbindungspartner.

30. Verfahren nach einem der Ansprüche 27 bis 29, dadurch gekennzeichnet, daß es weiterhin folgenden Schritt umfaßt:
g) man gibt in die zu prüfende Probe mindestens einen spezifischen Nebenbindungspartner.

31. Verfahren nach einem der Ansprüche 27 bis 30, dadurch gekennzeichnet, daß die Schritte a, e, f und g gleichzeitig oder nacheinander erfolgen.

32. Verfahren zur Dosierung eines Analyten in einer zu prüfenden Probe, dadurch gekennzeichnet, daß es die Schritte eines Nachweisverfahrens nach einem der Ansprüche 27 bis 31 und folgende Schritte umfaßt:
h) man mißt das von dem Indikatorreagenz gesendete Signal und
i) stellt eine Korrelation zwischen dieser Messung und der in der Probe vorliegenden Menge an Analyt her.

## Claims

1. Indicator reagent for the detection or assay of an analyte in a test sample, comprising a tracer which is attached to a specific bonding partner and is able to emit a signal when the specific bonding partner bonds to a complementary partner, characterized in that the tracer is a water-soluble conductive polymer.

2. Reagent according to Claim 1, characterized in that the conductive polymer is a homopolymer or copolymer formed by condensation of substituted derivatives of monomers selected from the group consisting of aniline, pyrrole, thiophene, acetylene, phenylene, naphthalene, carbazole, pyrene, pyrenylene vinylene, azulene, isothianaphthene, phenylene vinylene and thiophenol.

3. Reagent according to either of Claims 1 and 2, characterized in that the conductive polymer is formed from monomers substituted by one or more groups selected from carboxyl, sulpho, phenol, thiol, alcohol, aldehyde, amine, nitrile, amide, ketone, nitro and halogenated groups.

4. Reagent according to either of Claims 2 and 3, characterized in that one or more of the substituent groups is or are fixed directly to the monomer.

5. Reagent according to any one of Claims 2 to 4, characterized in that one or more of the substituent groups is or are fixed indirectly to the monomer.

6. Reagent according to either of Claims 2 and 4, characterized in that the water-soluble conductive polymer is poly(2-anilinecarboxylic acid).

7. Reagent according to either of Claims 2 and 4, characterized in that the water-soluble conductive polymer is poly(2-anilinesulphonic acid).

8. Reagent according to either of Claims 2 and 4, characterized in that the water-soluble conductive polymer is poly(3-thiophenecarboxylic acid).

9. Reagent according to either of Claims 2 and 4, characterized in that the water-soluble conductive polymer is poly(3-thiophenecarboxaldehyde).

10. Reagent according to any one of Claims 1 to 9, characterized in that it is in dry form.

11. Reagent according to any one of the preceding claims, characterized in that it is in combination with a solid support.

12. Reagent according to Claim 11, characterized in that it is in the form of a film.

13. Reagent according to any one of Claims 1 to 9, characterized in that it is in a form in which it is dissolved in an aqueous medium.

14. Reagent according to any one of the preceding claims, characterized in that the signal is an optical signal which can be detected or quantified by the eye.

15. Reagent according to any one of the preceding claims, characterized in that the signal is an optical signal which can be detected or quantified by an instrument.

16. Reagent according to Claim 15, characterized in that the instrument is able to detect or quantify infrared, visible or ultraviolet light absorption, refraction, diffraction or reflection.

17. Reagent according to any one of the preceding claims, characterized in that the specific bonding partner and its complementary partner are selected from the group consisting of antigens, haptens and antibodies, enzymes and substrates, receptors and ligands.

18. Reagent according to any one of the preceding claims, characterized in that the tracer is attached to the specific bonding partner by covalent bonding.

19. Reagent according to any one of Claims 1 to 17, characterized in that the tracer is attached to the specific bonding partner by passive adsorption.

20. Reagent according to any one of the preceding claims, characterized in that the tracer is attached to a principal specific bonding partner.

21. Reagent according to any one of Claims 1 to 19, characterized in that the tracer is attached to an auxiliary specific bonding partner.

22. Use of a soluble conductive polymer as a tracer capable of attaching itself to a specific bonding partner and able to be present in an indicator reagent according to any one of Claims 1 to 21.

23. Kit for the detection or assay of an analyte in a test sample, characterized in that it comprises at least one indicator reagent according to any one of Claims 1 to 21.

24. Kit according to Claim 23, characterized in that it additionally comprises a capture reagent.

25. Kit according to Claim 24, characterized in that the capture reagent is fixed to a solid phase.

26. Kit according to any one of Claims 23 to 25, characterized in that it additionally comprises at least one auxiliary specific bonding partner.

27. Method of detecting an analyte in a test sample, characterized in that it comprises the following steps:
a) introducing into the sample at least one indicator reagent according to any one of Claims 1 to 21;
b) allowing the indicator reagent to bond to the complementary partner of the specific bonding partner it contains;
c) separating, if appropriate, the free and bonded fractions of the indicator reagent;
d) detecting the indicator reagent and hence the presence of the analyte in the sample.

28. Method according to Claim 27, characterized in that it additionally comprises the following step:
e) introducing into the test sample a capture reagent, optionally fixed to a solid phase.

29. Method according to either of Claims 27 and 28, characterized in that it additionally comprises the following step:
f) if the specific bonding partner of the indicator reagent does not have the analyte as its complementary partner, introducing into the test sample at least one principal specific bonding partner.

30. Method according to any one of Claims 27 to 29, characterized in that it additionally comprises the following step:
g) introducing into the test sample at least one auxiliary specific bonding partner.

31. Method according to any one of Claims 27 to 30, characterized in that steps a, e, f and g proceed simultaneously or in sequence.

32. Method of assaying an analyte in a test sample, characterized in that it comprises the steps of a detection method according to any one of Claims 27 to 31 plus the following steps:
h) the signal emitted by the indicator reagent is measured and
i) a correlation is established between this measurement and the amount of analyte present in the sample.
